# EUROPEAN PATENT APPLICATION

(11) **EP 4 331 666 A1**
(43) Date of publication of application: **06.03.2024**
(21) Application number: 22795408.8
(22) Date of filing: 25.03.2022
(51) Int. Cl.: A61P 3/06, A61P 9/10, A61K 31/4164, A23L 33/175

(54) **COMPOSITION FOR IMPROVING BLOOD CHOLESTEROL LEVEL**

(30) Priority: 26.04.2021 JP 2021074318
(71) Applicant: Suntory Holdings Limited, Osaka 530-8203 (JP)
(72) Inventor: KATSUBE, Makoto, Soraku-gun, Kyoto 619-0284 (JP); WATANABE, Hiroshi, Soraku-gun, Kyoto 619-0284 (JP); MURAYAMA, Norihito, Soraku-gun, Kyoto 619-0284 (JP)
(74) Representative: Vossius & Partner Patentanwälte Rechtsanwälte mbB
(86) International application number: PCT/JP2022/014243
(87) International publication number: WO 2022/230490

(57) **Abstract**

The present invention aims to provide a composition for improving a blood cholesterol level and a method of improving a blood cholesterol level. The present invention relates to a composition for improving a human blood cholesterol level, which contains L-ergothioneine or a salt thereof as an active ingredient.

## Description

### TECHNICAL FIELD

The present invention relates to a composition for improving a human blood cholesterol level. The present invention relates to, for example, a method of improving a blood cholesterol level.

### BACKGROUND ART

Low-density lipoprotein (LDL) cholesterol and highdensity lipoprotein (HDL) cholesterol are lipids present in the human body. Excess LDL cholesterol is known to cause arteriosclerosis. In contrast, presumably, HDL cholesterol acts to collect excess cholesterol to prevent arteriosclerosis. Reducing the ratio of LDL cholesterol level to HDL cholesterol level (LDL/HDL ratio) in the blood and increasing the HDL cholesterol level in the blood are recommended to prevent or reduce arteriosclerosis.

Ergothioneine is an amino acid found in mushrooms and the like. L-ergothioneine is present in nature. According to Patent Literature 1, the triglyceride (neutral fat) level in plasma was lower in mice orally administered with ergothioneine than in mice not orally administered with ergothioneine.

### CITATION LIST

### - Patent Literature

Patent Literature 1: JP 2011-102286 A

### SUMMARY OF INVENTION

### - Technical Problem

However, Patent Literature 1 nowhere studies a substance that reduces the ratio of LDL cholesterol level to HDL cholesterol level (LDL/HDL ratio) in the blood. Patent Literature 1 is also silent about a substance having an action to increase the HDL cholesterol level in the blood.

The present invention aims to provide a composition for improving a blood cholesterol level. The present invention also aims to provide a method of improving a blood cholesterol level.

### - Solution to Problem

As a result of extensive studies to solve the above problem, the present inventors found that L-ergothioneine or a salt thereof has actions to improve cholesterol levels, such as an action to reduce the LDL/HDL ratio in the blood of humans.

Specifically, the present invention relates to, but is not limited to, a composition for improving a blood cholesterol level, a method of improving a blood cholesterol level, and the like described below.
(1) A composition for improving a human blood cholesterol level, containing L-ergothioneine or a salt thereof as an active ingredient.
(2) The composition according to (1) above for use in reducing a ratio of LDL cholesterol level to HDL cholesterol level, i.e., LDL/HDL ratio, in blood.
(3) The composition according to (1) or (2) above for use in increasing HDL cholesterol level in blood.
(4) The composition according to any one of (1) to (3) above for use in preventing arteriosclerosis.
(5) The composition according to any one of (1) to (4) above, wherein the composition is an oral composition.
(6) The composition according to any one of (1) to (5) above, wherein the composition is a food or beverage.
(7) The composition according to any one of (1) to (6) above, wherein an amount of L-ergothioneine or a salt thereof in the composition per adult daily intake is 2 to 50 mg in terms of L-ergothioneine.
(8) A method of improving a blood cholesterol level, including administering L-ergothioneine or a salt thereof to a human.
(9) Use of L-ergothioneine or a salt thereof for improving a blood cholesterol level in a human.

### - Advantageous Effects of Invention

The present invention can provide a composition for improving a human blood cholesterol level. The present invention can provide a method of improving a blood cholesterol level.

### BRIEF DESCRIPTION OF DRAWINGS

FIG. 1 is a graph showing changes in LDL/HDL ratio (Δ in LDL/HDL ratio) in the blood in a test food group and a control food group.
FIG. 2 is a graph showing changes in HDL cholesterol level (Δ in HDL cholesterol) in the blood in the test food group and the control food group.

### DESCRIPTION OF EMBODIMENTS

The composition for improving blood cholesterol levels of the present invention is a composition for improving human blood cholesterol levels. The composition for improving blood cholesterol levels of the present invention contains L-ergothioneine or a salt thereof as an active ingredient. Hereinafter, the composition for improving blood cholesterol levels of the present invention is sometimes simply referred to as "the composition of the present invention".

L-ergothioneine is one of the amino acids.

The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopiae* var. *citrinopileatus*) belonging to the genus *Pleurotus* of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus*), shiitake (binomial name: *Lentinula edodes*), hen-of-the-wood (binomial name: *Grifola Frondosa*), reishi mushuroom (binomial name: *Ganoderma lucidum*), lion's mane mushroom (binomial name: *Hericium erinaceus*), Yanagimatsutake (binomial name: *Agrocybe aegerita*), girolle (binomial name: *Cantharellus cibarius*)*,* porcini (binomial name: *Boletus edulis*), and common morel (binomial name: *Morchella esculenta*). When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form.

L-ergothioneine or a salt thereof is present in natural products and food and beverages, and it is a compound that has been consumed. Thus, continuous ingestion of L-ergothioneine or a salt thereof, for example, is less likely to cause problems in terms of safety.

The composition of the present invention is used to improve human blood cholesterol levels. In the present invention, improvement in blood cholesterol levels is preferably a reduction in the ratio of LDL cholesterol level to HDL cholesterol level (cholesterol concentration) (LDL/HDL ratio) in the blood and/or an increase in the HDL cholesterol level in the blood.

As described later in Examples, the HDL cholesterol level in the blood was increased by ingestion of L-ergothioneine compared to no ingestion of L-ergothioneine. The ratio of LDL cholesterol level to HDL cholesterol level (LDL/HDL ratio) in the blood was reduced by ingestion of L-ergothioneine compared to no ingestion of L-ergothioneine.

Thus, L-ergothioneine or a salt thereof has an action to improve blood cholesterol levels in humans and can be used as an active ingredient for improving blood cholesterol levels.

In one embodiment, the composition of the present invention can be used to reduce the ratio of LDL cholesterol level to HDL cholesterol level (LDL/HDL ratio) in the blood and/or to increase the HDL cholesterol level in the blood. In a preferred embodiment, the composition of the present invention can be used to reduce the ratio of LDL cholesterol level to HDL cholesterol level (LDL/HDL ratio) in the blood and to increase the HDL cholesterol level in the blood.

In one embodiment, the composition of the present invention is useful in preventing or ameliorating a condition or disease which can be effectively prevented or ameliorated by improving blood cholesterol levels. An effect of preventing or ameliorating a condition or disease such as arteriosclerosis, myocardial infarction, angina pectoris, cerebral infarction, or fatty liver disease can be achieved by improving blood cholesterol levels. In one embodiment, the composition of the present invention can be used to prevent or ameliorate a condition or disease such as arteriosclerosis, myocardial infarction, angina pectoris, cerebral infarction, or fatty liver disease. In one embodiment, the composition of the present invention is suitably used to prevent arteriosclerosis.

Preventing a condition or disease encompasses preventing the onset, delaying the onset, reducing the incidence, reducing the onset risk, and the like. Ameliorating a condition or disease encompasses helping a subject recover from a condition or disease, alleviating a symptom of a condition or disease, favorably changing a symptom of a condition or disease, delaying or preventing the progress of a condition or disease, and the like.

The composition of the present invention is applicable for therapeutic use (medical use) and non-therapeutic use (non-medical use). The "non-therapeutic" is a concept that does not include medical activities, i.e., a concept that does not include methods of surgery, therapy, or diagnosis of humans.

The composition for improving blood cholesterol levels of the present invention can be provided as an agent or the like, for example, but it is not limited thereto. The agent can be directly provided as a composition or can be provided as a composition containing the agent. In one embodiment, the composition for improving blood cholesterol levels of the present invention can also be referred to as an agent for improving blood cholesterol levels.

In order to sufficiently obtain the effect of the present invention, preferably, the composition of the present invention is an oral composition. The oral composition may be a food or beverage, an oral pharmaceutical product, or an oral quasi-pharmaceutical product, preferably a food or beverage or an oral pharmaceutical product, still more preferably a food or beverage.

The composition of the present invention may contain optional additives and optional components in addition to L-ergothioneine or a salt thereof, as long as the effect of the present invention is not impaired. Such additives and components can be selected according to the composition form or the like. Those that can be generally used in foods, beverages, pharmaceutical products, quasi-pharmaceutical products, and the like can be used. When the composition of the present invention is provided as a food or beverage, a pharmaceutical product, a quasi-pharmaceutical product, or the like, the production method is not limited, and any common method can be used for production.

For example, when the composition of the present invention is provided as a food or beverage, various types of food or beverages can be provided by adding a component usable in food or beverages (e.g., a food material or an optional food additive) to L-ergothioneine or a salt thereof. Non-limiting examples of the food or beverages include general foods and beverages, health foods, health beverages, foods with function claims, foods for specified health uses, dietary supplements, and foods and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The administration form of pharmaceutical products or quasi-pharmaceutical products may be oral or parenteral. Oral administration is preferred in order to obtain the effect of the present invention more sufficiently. When the composition of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion.

The amount of L-ergothioneine or a salt thereof in the composition of the present invention is not limited and can be set according to the composition form and the like.

The amount of L-ergothioneine or a salt thereof, for example, in terms of L-ergothioneine, in the composition of the present invention is preferably 0.0001 wt% or more, more preferably 0.001 wt% or more and is preferably 90 wt% or less, more preferably 50 wt% or less. In one embodiment, the amount of L-ergothioneine or a salt thereof in terms of L-ergothioneine in the composition is preferably 0.0001 to 90 wt%, more preferably 0.001 to 50 wt%. In one embodiment, when the composition of the present invention is provided as a food or beverage, a pharmaceutical product, a quasi-pharmaceutical product, or the like, preferably, the amount of L-ergothioneine or a salt thereof is in the above ranges.

In the case of L-ergothioneine, the amount in terms of L-ergothioneine or an expression similar thereto refers to the amount of L-ergothioneine. In the case of a salt of L-ergothioneine, the amount in terms of L-ergothioneine or an expression similar thereto refers to a value obtained by multiplying the molar number of the salt by the molecular weight of L-ergothioneine.

The composition of the present invention can be fed or administered by a method appropriate to the composition form. In order to more sufficiently obtain the effect of the present invention, preferably, the composition of the present invention is orally fed (orally administered). The intake (administration amount) of the composition of the present invention is not limited as long as it is an amount that provides an effect of improving blood cholesterol levels and may be appropriately set according to the administration form, administration method, body weight of a subject, and the like.

In one embodiment, when orally feeding or administering the composition of the present invention to a human (adult) as a subject, the intake of L-ergothioneine or a salt thereof in terms of L-ergothioneine per day is preferably 2 mg or more, more preferably 5 mg or more, still more preferably 10 mg or more, and is preferably 50 mg or less, more preferably 25 mg or less, still more preferably 20 mg or less. In one embodiment, when orally feeding or administering the composition of the present invention to a human (adult) as a subject, the intake of L-ergothioneine or a salt thereof in terms of L-ergothioneine per day is preferably 2 to 50 mg, more preferably 5 to 25 mg, still more preferably 5 to 20 mg, particularly preferably 10 to 20 mg. Preferably, the above amount of the composition is fed or administered in one or more portions per day, for example, in one portion or several portions (for example, two or three portions) per day. In one embodiment of the present invention, the composition of the present invention may be an oral composition for feeding or administering the above amount of L-ergothioneine or a salt thereof per 60 kg body weight per day to a human.

In one embodiment, when the composition of the present invention is parenterally administered to a human (adult), the administration amount of L-ergothioneine or a salt thereof in terms of L-ergothioneine per day is, for example, preferably 2 to 50 mg, more preferably 5 to 25 mg, still more preferably 5 to 20 mg, particularly preferably 10 to 20 mg.

In one embodiment, preferably, the above amount of L-ergothioneine or a salt thereof is fed or administered to a human (adult), per 60 kg body weight per day.

In one embodiment, preferably, the amount of L-ergothioneine or a salt thereof in the composition of the present invention per adult daily intake is 2 to 50 mg in terms of L-ergothioneine. The amount of L-ergothioneine or a salt thereof in the composition of the present invention per adult daily intake is more preferably 5 to 25 mg, still more preferably 5 to 20 mg, particularly preferably 10 to 20 mg in terms of L-ergothioneine.

Continuous feeding or administration of L-ergothioneine or a salt thereof is likely to enhance the effect of improving blood cholesterol levels. Thus, in a preferred embodiment, the composition of the present invention is continuously fed or administered. In one embodiment of the present invention, the composition of the present invention is continuously fed or administered preferably for at least one week, more preferably for at least two weeks.

The subject to be fed or administered with the composition of the present invention (subject to administration) is a human.

The subject to be fed or administered with the composition of the present invention may be one needing or wanting to improve blood cholesterol levels. Examples of the subject to administration include a human needing or wanting to reduce the ratio of LDL cholesterol level to HDL cholesterol level (LDL/HDL ratio) in the blood and a human needing or wanting to increase the HDL cholesterol level in the blood. In the present invention, examples of the subject to administration also include a human needing or wanting to prevent or ameliorate a condition or disease such as arteriosclerosis, myocardial infarction, angina pectoris, cerebral infarction, or fatty liver disease. In one embodiment, the composition of the present invention is suitably applicable to a human needing or wanting to prevent or reduce arteriosclerosis (particularly to prevent arteriosclerosis). In one embodiment, examples of the subject to administration include a human with the condition or disease described above, for example. In one embodiment, the subject to administration of the composition of the present invention may be a healthy person. The composition of the present invention can be used on a healthy person, for example, for purposes such as preventing a condition or disease which can be effectively prevented or ameliorated by improving blood cholesterol levels.

The composition of the present invention may be labeled with a function claim based on improvement in blood cholesterol levels. For example, the composition of the present invention may be labeled with a function claim such as "preventing arteriosclerosis".

In one embodiment of the present invention, preferably, the composition of the present invention is a food or beverage labeled with such a function claim. The label may be one indicating use of the composition for obtaining the function. The label may be directly attached to the composition or may be attached to a container or a package of the composition.

The present invention encompasses the following method and use:
a method of improving a blood cholesterol level, including feeding or administering L-ergothioneine or a salt thereof to a human; and
use of L-ergothioneine or a salt thereof for improving a blood cholesterol level in a human.

The above method is a method of improving blood cholesterol levels in humans. Feeding or administering L-ergothioneine or a salt thereof to a subject can improve blood cholesterol levels. Preferably, L-ergothioneine or a salt thereof is orally fed or administered.

The method may be therapeutic or non-therapeutic. The use may be therapeutic or non-therapeutic.

The above method may be a method of reducing the ratio of LDL cholesterol level to HDL cholesterol level (LDL/HDL ratio) in the blood and/or a method of increasing the HDL cholesterol level in the blood. The above use may be use of L-ergothioneine or a salt thereof for reducing the ratio of LDL cholesterol level to HDL cholesterol level (LDL/HDL ratio) in the blood and/or for increasing the HDL cholesterol level in the blood.

In the method and use, L-ergothioneine or a salt thereof, preferred embodiments thereof, and the like are as described above for the composition for improving blood cholesterol levels of the present invention. In the method and use, preferably, L-ergothioneine or a salt thereof is fed or administered to a subject one or more times per day, for example, one to several times (e.g., two to three times) per day. In one embodiment, L-ergothioneine or a salt thereof can be used to prevent or ameliorate a condition or disease such as arteriosclerosis, myocardial infarction, angina pectoris, cerebral infarction, or fatty liver disease by improving blood cholesterol levels.

In the method and use, L-ergothioneine or a salt thereof is simply used in an amount (effective amount) that provides the effect of improving blood cholesterol levels. Preferred administration amounts of L-ergothioneine or a salt thereof, preferred subjects to administration, and the like are as described above for the composition for improving blood cholesterol levels of the present invention. L-ergothioneine or a salt thereof may be directly fed or administered or may be fed or administered as a composition containing L-ergothioneine or a salt thereof. For example, the composition of the present invention may be fed or administered.

L-ergothioneine or a salt thereof can also be used to produce a food or beverage, a pharmaceutical product, a quasi-pharmaceutical product, or the like for use in improving blood cholesterol levels. In one embodiment, the present invention also encompasses use of L-ergothioneine or a salt thereof to produce a composition for improving blood cholesterol levels.

The present invention also encompasses L-ergothioneine or a salt thereof for use in improving blood cholesterol levels in humans.

### EXAMPLES

The present invention is described in further detail below with reference to examples, but the scope of the present invention is not limited thereto.

### <Example 1>

### (Blood cholesterol evaluation study on human)

In order to evaluate the influence of supplements containing ergothioneine on human blood cholesterol levels, a placebo-controlled, randomized, double-blind, parallelgroup study was conducted on subjects including male and female adults (46 subjects in a test food group; 46 subjects in a control food group; 92 subjects in total).

In this study, one capsule containing 20 mg ergothioneine (test food) or one capsule not containing ergothioneine (control food) was fed per day for four weeks. The blood cholesterol levels of the subjects were measured for pre-testing before starting the study (before starting ingestion of the test food or control food). After ingestion of the test food or control food for four weeks, the blood cholesterol levels of the subjects were measured again (testing on week 4). The cholesterol measured includes HDL cholesterol and LDL cholesterol.

### (Food for evaluation)

Two types of food for evaluation, which were indistinguishable in appearance, flavor, and the like, were used.
· Test food: capsule containing a test substance (20 mg L-ergothioneine)
· Control food: capsule containing no test substance

Each type of food for evaluation contained raw materials such as dextrin, hydroxypropyl cellulose, carrageenan, potassium chloride, and titanium oxide, in addition to the test substance. The control food was produced using the same raw materials used in the test food, except that the test substance (L-ergothioneine) was not added.

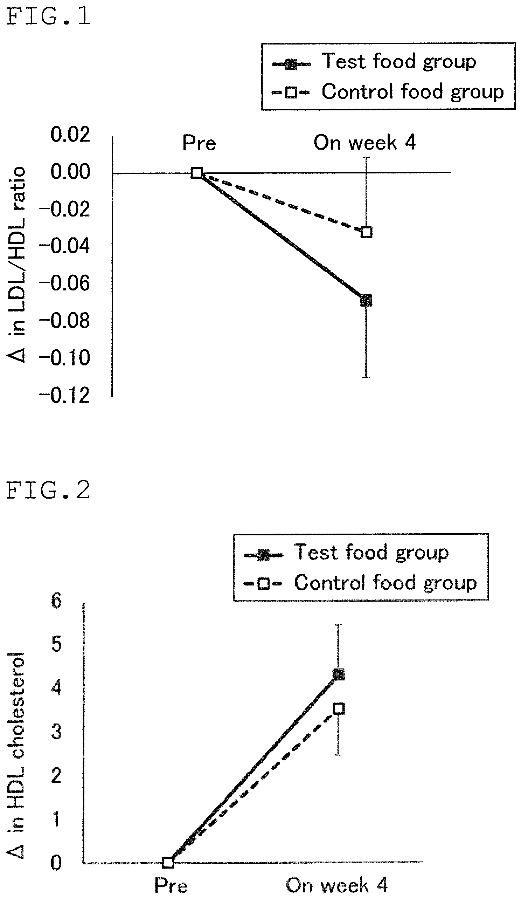

The ratio of LDL cholesterol level to HDL cholesterol level (LDL/HDL ratio) in the blood was determined for each subject from the measurements (mg/dL) of HDL cholesterol and LDL cholesterol. The average LDL/HDL ratio of the subjects in the test food group was regarded as the LDL/HDL ratio of the test food group. The average LDL/HDL ratio of the subjects in the control food group was regarded as the LDL/HDL ratio of the control food group. For each group, the amount of changes in LDL/HDL ratio (Δ in LDL/HDL ratio) from the time of pre-testing was determined by subtracting the LDL/HDL ratio at the time of pre-testing from the LDL/HDL ratio at the time of testing on week 4 ((LDL/HDL ratio at the time of testing on week 4) - (LDL/HDL ratio at the time of pre-testing)). The amount of changes in LDL/HDL ratio (Δ in LDL/HDL ratio) at the time of pre-testing was set to 0. FIG. 1 shows the results.

FIG. 1 is a graph showing changes in the LDL/HDL ratio (Δ in LDL/HDL ratio) in the blood in the test food group and the control food group. In FIG. 1 and FIG. 2, ■ (black square) indicates the test food group and □ (white square) indicates the control food group. On the horizontal axis, "Pre" indicates at the time of pre-testing and "On week 4" indicates at the time of testing on week 4.

The results show that the LDL/HDL ratio in the blood was reduced more in the test food group than in the control food group.

For each subject, Δ value (the amount of changes in HDL cholesterol level) (mg/dL) was determined by subtracting the HDL cholesterol measurements at the time of pre-testing from the HDL cholesterol measurements at the time of testing on week 4 ((measurements at the time of testing on week 4) - (measurements at the time of pre-testing)). The average Δ value of the subjects in the test food group was regarded as the amount of changes in HDL cholesterol level (Δ in HDL cholesterol) of the test food group. The average Δ value of the subjects in the control food group was regarded as the amount of changes in HDL cholesterol level (Δ in HDL cholesterol) of the control food group. The amount of changes in HDL cholesterol level at the time of pre-testing was set to 0. FIG. 2 shows the results.

FIG. 2 is a graph showing changes in HDL cholesterol level (Δ in HDL cholesterol) in the blood in the test food group and the control food group.

According to the results, the HDL cholesterol level in the blood was increased more in the test food group than in the control food group.

The above results show that L-ergothioneine or a salt thereof has an action to improve blood cholesterol levels in humans.

## Claims

1. A composition for improving a human blood cholesterol level, comprising:
L-ergothioneine or a salt thereof as an active ingredient.

2. The composition according to claim 1 for use in reducing a ratio of LDL cholesterol level to HDL cholesterol level, i.e., LDL/HDL ratio, in blood.

3. The composition according to claim 1 or 2 for use in increasing HDL cholesterol level in blood.

4. The composition according to any one of claims 1 to 3 for use in preventing arteriosclerosis.

5. The composition according to any one of claims 1 to 4,
wherein the composition is an oral composition.

6. The composition according to any one of claims 1 to 5,
wherein the composition is a food or beverage.

7. The composition according to any one of claims 1 to 6,
wherein an amount of L-ergothioneine or a salt thereof in the composition per adult daily intake is 2 to 50 mg in terms of L-ergothioneine.

8. A method of improving a blood cholesterol level, comprising:
administering L-ergothioneine or a salt thereof to a human.

9. Use of L-ergothioneine or a salt thereof for improving a blood cholesterol level in a human.
